# EUROPEAN PATENT APPLICATION

(11) **EP 1 808 163 A1**
(43) Date of publication of application: **18.07.2007**
(21) Application number: 05258010.7
(22) Date of filing: 22.12.2005
(51) Int. Cl.: A61K 9/20, A61K 9/16, A61K 9/14, A61K 31/216

(54) **Compressed solid dosage forms with drugs of low solubility and process for making the same**

(71) Applicant: TEVA PHARMACEUTICAL INDUSTRIES LTD., 49131 Petah Tiqva (IL)
(72) Inventor: Zalit, Ilan, Rosh Ha Ayin (IL); Kopel, Mira, Katzir 37861 (IL)
(74) Representative: Gallagher, Kirk James

(57) **Abstract**

One of the objects of the present invention is directed to a process of preparing a pharmaceutical formulation of a drug of low aqueous solubility, comprising (A) fixing the drug in a strong matrix comprising at least one at least partially amorphous sugar to obtain a sugar-drug matrix; and (B) milling the sugar-drug matrix to obtain a milled sugar-drug matrix as the pharmaceutical formulation. The invention also provides the pharmaceutical formulation prepared by the process.

## Description

The present invention concerns a process to promote dissolution of poorly water soluble drugs in order to achieve rates of dissolution and absorption comparably similar to, or even faster than, the recent generation of technologies and products in the prior art.

### BACKGROUND OF THE INVENTION

When solid dosage forms are taken orally, in many cases, the drug must dissolve in aqueous gastrointestinal fluids, for example in the patient's stomach, before the drug can exert a therapeutic effect. A recurring problem with compressed solid oral dosage forms, such as tablets, capsules and caplets (i.e., capsule-shaped tablets), is that the dissolution rate of some drugs from the dosage form limits their biological availability. This problem arises from the fact that many drugs are small organic molecules with low solubility in aqueous fluids. There are several ways to address the solubility problem of poorly soluble drugs

For example, the drug itself can be modified. The physical form of the drug can be manipulated by various techniques to optimize the rate at which the drug dissolves. Of these techniques, the one most relevant to the present invention is particle size reduction. The rate of dissolution of a solid may often depend upon the surface area that is exposed to the dissolving medium and since the surface area of a given mass of a substance is generally inversely proportional to the substance's particle size, reducing the particle size of a powder or granular substance may increase its dissolution rate.

Where it is effective, particle size reduction often increases the dissolution rate of a particulate solid by increasing the surface area that is exposed to the dissolving medium. However, particle size reduction is not always effective at increasing the dissolution rate of a drug from a compressed solid dosage form. Many hydrophobic drugs have a strong tendency to agglomerate during the dosage form manufacturing process into larger particles with an overall decrease in effective surface area. Remington: The Science and Practice of Pharmacy, 20th ed. 656, 657 (A.R. Gennaro, ed., Lippincott Williams & Wilkins: Philadelphia 2000), incorporated by reference herein, contains a more thorough discussion of the concept of "effective surface area" and the effect of particle size on dissolution. A drug that has ostensibly been milled to a fine particle size will sometimes display dissolution characteristics of a larger particle due to agglomeration or similar effect.

Recently in the field of the pharmaceutical industry pharmaceutical companies have been trying to develop a new generation of products with increased surface area of the drug particles with concomitant increased dissolution rates and absorption. This is achieved by a variety of methods including production of a formulation with nano particles (i.e., with diameters less than 1µm), creating stable nano-particles of amorphous material stabilized by hydrophilic polymer, and stabilizing small particles by making a complex with surface active agent or agents via melting of the two substances. In general the new approaches of the pharmaceutical industry face two major barriers. The first barrier is the technical /mechanical limitation of breaking the drug particles into the size of nano particles. The second barrier is the stabilization of these small particles of the drug substance in the dosage form, whether they are in crystalline or amorphous form.

### SUMMARY OF THE INVENTION

The present invention provides a process for making a pharmaceutical formulation of an active pharmaceutical ingredient (API), i.e., drug, having low aqueous solubility, the process comprising
(A) fixing the drug in a strong matrix comprising at least one at least partially amorphous sugar, which sugar is preferably substantially amorphous or entirely amorphous, to obtain a sugar-drug matrix; and
(B) milling, preferably intensely, the sugar-drug matrix to obtain a milled sugar-drug matrix as the pharmaceutical formulation.

The fixing step, i.e., step (A), of the above process of the invention is performed by heating a mixture of the drug, optionally pre-mixed or pre-granulated with at least one inactive excipient, and the at least one at least partially amorphous sugar followed by cooling. Alternatively, the fixing step of the above process of the invention is performed by heating a mixture of the drug, optionally pre-mixed or pre-granulated with inactive excipient, and at least one sugar followed by cooling, wherein the at least one sugar is converted to the at least one at least partially amorphous sugar. Preferably, the heating is accompanied with mixing of the drug and the sugar. The "sugar-drug matrix" obtained in the fixing step, i.e., step (A), of the above process preferably is a homogeneous dispersion of particles of the drug in the strong matrix. However, the sugar-drug matrix may also be only partially homogeneous without departing from the spirit of the invention but preferably the sugar-drug matrix obtained in the fixing step will be substantially homogeneous which is understood to be of an even consistency by visual inspection. Step (B) involves milling, preferably intensely, the "sugar-drug matrix" obtained in step (A), e.g., by using a comminuting mill exemplified by a Fitzmill^{™} Communitor (Fitzpatrick "Knives" forward).

In a first embodiment of the process of the invention for making the pharmaceutical formulation of the drug having low aqueous solubility, step (A) is performed as step (a) below and step (B) is performed as step (b) below, such that the embodiment comprises
(a) mixing while heating the drug, having low aqueous solubility, e.g., fenofibrate 145 mg, with the following excipient(s) to form a sugar-drug matrix (wherein the drug can be a formulated particulate composition which has been previously compounded):
   (i) at least one at least partially amorphous sugar, which sugar is preferably substantially amorphous or entirely amorphous, in a Sugar-Drug ratio of from about 1.5:1 to about 10:1, preferably from about 2:1 to about 8:1 and more preferably from about 3:1 to about 6:1, wherein the amorphous sugar was prepared for example, by heating sucrose, glucose and water to a high temperature to form a mix and then cooling the mix,
   (ii) optionally a surface active agent, e.g., polysorbate, glycerol monostearate and, preferably, sodium lauryl sulfate (SLS), in an amount of from about 1 to about 5 weight % of the final pharmaceutical formulation, and
   (iii) optionally, a dispersing agent, e.g., poloxamer, polysorbate, and preferably polyvinylpyrrolidone (PVP), in an amount of from about 1 to about 10 weight % of the final pharmaceutical formulation; and
(b) milling, preferably intensely, the sugar-drug matrix to obtain a milled sugar-drug matrix as the pharmaceutical formulation, wherein the milling is optionally repeated until the "drug-sugar matrix" achieves the desired dissolution property, and wherein the sugar-drug matrix is about 10% to about 99 weight%, preferably from about 30% to about 95 weight%, and more preferably from about 65% to about 90 weight%, of the final pharmaceutical formulation.

In a second embodiment of the process of the invention for making the pharmaceutical formulation of the drug having low aqueous solubility, step (A) is performed by conducting steps (a')-(c') described below and step (B) is performed by conducting step (d') below, such that the second embodiment comprises:
(a') blending the drug having low aqueous solubility in a particulate form, the at least one at least partially amorphous sugar in powder form, the optional surface active agent (e.g., polysorbate, glycerol monostearate and, preferably, sodium lauryl sulfate) and the optional dispersing agent (e.g., poloxamer, polysorbate and, preferably, polyvinylpyrrolidone), wherein the drug can be a formulated particulate composition which has been previously compounded;
(b') heating the blend with mixing, preferably to a temperature ranging from about 50° C to about 200° C and, more preferably from about 70° C to about 120° C, until a "smooth" mixture is obtained, wherein the temperature, time and intensity of mixing should be controlled, based on the mixing instrumentation used, to obtain the "smooth" mixture;
(c') cooling the "smooth" mixture to room temperature or below room temperature to obtain a sugar-drug matrix; and
(d') milling, preferably intensely, the sugar-drug matrix obtained in step (c') optionally with a glidant, e.g., by using a comminution mill exemplified by a Fitzmill^{™} Communitor ("Knives" forward) from The Fitzpatrick Company, or any similarly intensive milling machinery, to obtain a milled sugar-drug matrix as the pharmaceutical formulation, wherein the milled sugar-drug matrix comprises a powder.

The second embodiment of the process of the invention can optionally further comprise re-working the milled sugar-drug matrix as shown below:
(e') making a blend with a procedure comprising the following steps:
   (e'1) heating the milled sugar-drug matrix with mixing, preferably to a temperature ranging from about 50° C to about 200° C and, more preferably from about 70° C to about 120° C, until a "smooth" mixture is obtained;
   (e'2) cooling the "smooth" mixture to room temperature or below room temperature to obtain a cooled matrix;
   (e'3) milling, preferably intensely, the cooled matrix obtained in step (e'2), e.g., by using a comminuting mill exemplified by a Fitzmill^{™} Communitor ("knives" forward) from The Fitzpatrick Company, or any similarly intensive milling machinery, to obtain a milled matrix as a blend containing a powder; and
   (e'4) optionally repeating steps (e'1) to (e'3) until the powder in the milled matrix reaches a desired dissolution profile;
(f') optionally adding other pharmaceutically acceptable excipients, such as diluents, disintegrants, lubricants and glidants, to the blend obtained from step (d') or (e') in order to improve the handling and/or compression of the blend; and
(g') tabletting the blend or filling the blend into capsules or sachets.

The present invention also provides a pharmaceutical formulation of the drug having low aqueous solubility prepared by anyone of the embodiments of the process of the invention.

### BRIEF DESCRIPTION OF THE DRAWINGS

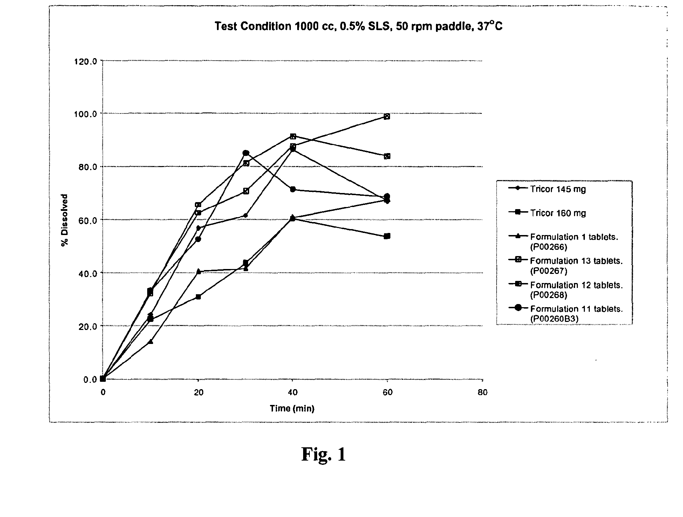

Fig. 1 shows the dissolution data of fenofibrate tablets having a medium content of at least partially amorphous sugar (Formulation 11) and fenofibrate tablets having a high content of a different type of at least partially amorphous sugar (Formulation 12) of the invention compared with that of commercially available fenofibrate tablets (Tricor 145 mg and Tricor 160 mg), fenofibrate tablets made by conventional wet granulation (Formulaion 1), and fenofibrate tablets made by using fenofibrate and crystalline sugar (Formulation 13).

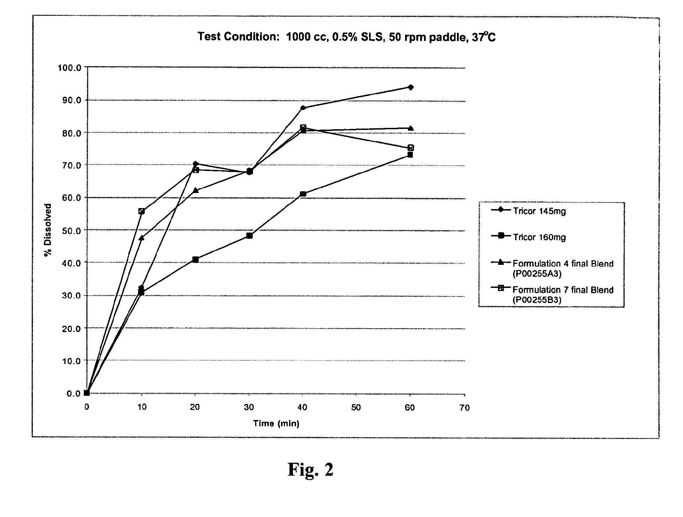

Fig. 2 shows the dissolution data of two fenofibrate blends having a high content of at least partially amorphous sugar (Formulations 4 and 7) according to the invention compared with that of commercially available fenofibrate formulations (Tricor 145 mg and Tricor 160 mg).

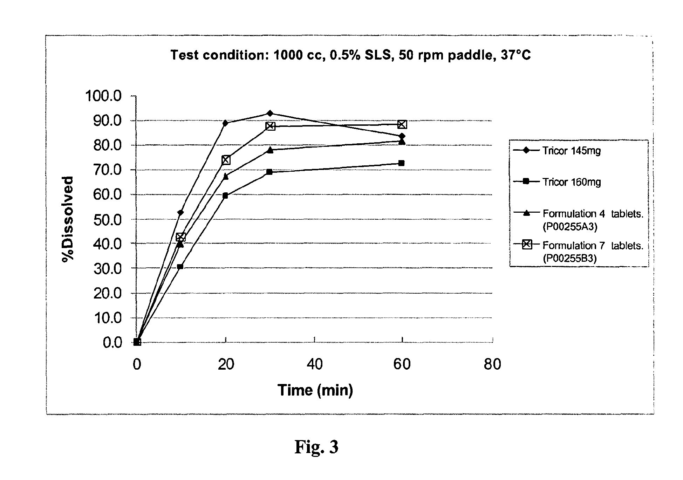

Fig. 3 shows the dissolution data of fenofibrate tablets 145 mg made with blends having a high ratio of sugar to drug in the "sugar-drug matrix" (Formulations 4 and 7) prepared according to the invention compared with that of the commercially available fenofibrate tablets (Tricor 145 mg and Tricor 160 mg).

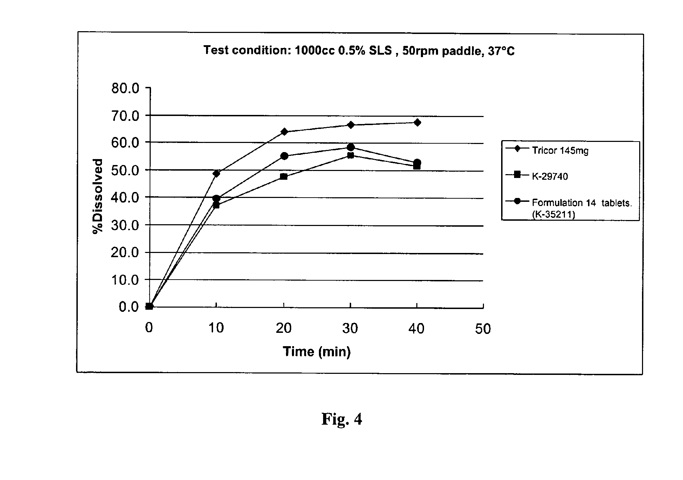

Fig. 4 shows the dissolution data of fenofibrate tablets 145 mg having a high ratio of sugar to drug in the "sugar-drug matrix" using a "formulated granulate of fenofibrate" as the source of the "drug" in the process of preparing the "sugar-drug matrix" according to the invention compared with the dissolution data of the commercially available Tricor 145 mg and Formulation K-29740 ( bioequivalent to Tricor 160 mg, K-29740 being not a formulation of the invention).

### DETAILED DESCRIPTION OF THE INVENTION

A drug has "low aqueous solubility" or is "poorly soluble in water" if the water solubility of the un-ionized form of the drug is less than about 1% by weight, and typically less than about 0.1% or 0.01% by weight. Such drugs include fenofibrate, bicalutamide,, atorvastatin, fluvastatin, , simvastatin, , paclitaxel, aripiprazole, glyburide, ezetimibe, oxcarbazepine , meloxicam, celecoxib, rofecoxib, valdecoxib and raloxifene.

The term "at least partially amorphous sugar" means that the sugar is partly or purely amorphous. The term "at least partially amorphous sugar" encompasses situations where part of the sugar used is crystalline, either due to incomplete conversion from crystalline to amorphous form or due to re-crystallization of a portion of an amorphous sugar. A sugar is "substantially amorphous" if the sugar is about 60-100 weight % such as about 60-95 weight %, more preferably about 80-99 weight %, and even more preferably about 90-99 weight %, amorphous. Formulations of the invention can be made from amorphous sugar that has been prepared without using glucose.

The term "strong matrix comprising at least one at least partially amorphous sugar" refers to a dense and strong material comprising at least one at least partially amorphous sugar and having physical characteristics, such as rigidity, hardness and denseness, very similar to that of boiled sugar candy or hard sugar candy.

The term "milling" is used generically to reflect methods of particle size reduction such as grinding of many types.

In the process of the invention, the term "'smooth" mixture' means that drug particles are evenly distributed in the sugar matrix and that the drug particles do not form lumps or agglomerates in the sugar-drug matrix.

In the pharmaceutical formulation of the drug of low aqueous solubility according to the invention, it is important to control and define the weight ratio between the at least one at least partially amorphous sugar and the drug in the "sugar-drug matrix" well. Preferably, it is also important to control and define the weight % of the "sugar-drug matrix" in the final formulation.

Generally, a high ratio of the at least one partially amorphous sugar to the drug in the "sugar-drug matrix" would be preferred, while the weight % of the "sugar-drug matrix" in the final formulation should be as small as possible. The extent to which the two parameters can be adjusted is dependent on the maximum allowed size of the dosage form, e.g., 2 grams for capsules or tablets, that must be swallowed, and the dose to be administered. For example, the ratio of the at least one at least partially amorphous sugar, and drug of low aqueous solubility can range from about 1:10 to about 1000:1 1 such as from about 1:1 to about 600:1, or from about 5:1 to about 300:1, or from about 10:1 to about 100:1, and the weight % of the "sugar-drug matrix" in the final formulation can range from about 0.5% to about 99.5% such as from about 5% to about 99%. For instance, for drugs generally used at a relatively high dose ( e.g. fenofibrate 145 mg), the weight ratio of the at least one at least partially amorphous sugar : drug can range from about 1.5:1 to about 10:1, preferably from about 2:1 to about 8:1 and more preferably from about 3:1 to about 6:1, and the weight % of the "sugar-drug matrix" in the final formulation can range from about 10 to about 99 weight %, preferably, from about 30 to about 95 weight % and, more preferably, from about 65 to about 90 weight %. On the other hand, for drugs generally used at a relatively low dose (e.g. glyboride 1 mg), the weight ratio of the at least one at least partially amorphous sugar:drug can be from about 1.5:1 to about 500:1, preferably from about 5:1 to about 50:1 and more preferably from about 10:1 to about 25:1, and the weight % of the "sugar-drug matrix" in the final formulation can range from about 10 to about 90 weight %, preferably, from about 15 to about 60 weight % and, more preferably, from about 20 to about 40 weight %.

In preparing formulations of the present invention with a specified dose of 145 mg, an example of using a relatively high weight ratio of the at least one at least partially amorphous sugar to drug in the final formulation is about 6:1 to about 4:1; an example of a medium weight ratio of the at least one at least partially amorphous sugar to drug in the final formulation is about 3:1 to about 4:1, e.g., about 3.4:1; and an example of a relatively low weight ratio of the at least one at least partially amorphous sugar to drug in the final formulation is about 0.5:1 to less than about 3:1, e.g., about 0.66:1, about 1.5:1, and about 2.6:1. In the formulation of the present invention with a specified dose of 145 mg, an example of a relatively high weight % of the "drug-sugar matrix" in the final formulation is about 65 weight% to about 95 weight%, e.g., about 78 weight%; and an example of a relatively low weight% of the "drug-sugar matrix" in the final formulation is about 10 weight% to less than about 65 weight%, e.g., about 20 weight%, about 23.4 weight%, about 40 weight%, about 42.5 weight%, about 58 weight% and about 60 weight%.

The solubility of the drug obtained by this process can be controlled by controlling the temperature, mixing intensity and/or mixing time of the heated mass. All these parameters enable controlling the geometry and "architecture" of the resultant sugar-drug matrix, and consequently may control the extent to which the milling of the sugar-drug matrix effects the solubility of the drug particles.

The process of the invention can involve two main operations: 1) the formation of a homogeneous dispersion of the drug particles in a strong sugar matrix, and 2) strong milling of the sugar-drug matrix. These two operations result in fine particles, which yield good dissolution and consequently good absorption.

Two main parameters are involved in the first operation of the process of the invention: the mixing temperature and mixing time of the "hot mixing stage", e.g., step (a) of the first embodiment or step (b') of the second embodiment, of the drug and the at least one at least partially amorphous sugar. When the mixing temperature is below the melting point of the drug, no melting of the drug particles should occur and the mixing of the drug and sugar results in good dispersion of the drug particles in the sugar-drug matrix. When the mixing temperature is above the melting point of the drug, at least a portion of the drug particles in the heated mass should melt or be deformed (depending on the mixing temperature) resulting in even better dispersion of the drug in the sugar-drug matrix. For any given mixing temperature, longer mixing time of the heated mass should improve the dispersion of the drug in the final sugar-drug matrix. A third parameter which may have an effect on the final dispersion of the drug in the drug-sugar matrix is the mixing of the drug with the at least one at least partially amorphous sugar before the heating stage, e.g., step (a') of the second embodiment, or any optional mixing of the drug and the at least one at least partially amorphous sugar before step (a) of the first embodiment. It is assumed that good dispersion of the drug particles in the mixture of the drug and the at least one at least partially amorphous sugar before heating can further improve the distribution of the drug particles in the sugar-drug matrix at the heating stage and consequently in the final "sugar-drug matrix". An example for the procedure for pre-mixing the drug and sugar comprises: I) direct mixing of the drug with the at least one at least partially amorphous sugar (with or without the addition of the surface active agent and dispersing agent, II) applying any other conventional or non-conventional granulation process which can improve the dispersion of drug particles to the product of step I) to form a pregranulated powder, and followed by III) using the pregranulated powder as a "drug source" in step (A) of the general process of the invention, step (a) of the first embodiment, or step (b') of the second embodiment.

The second operation of the above process involves strong milling of the final rigid sugar-drug matrix resulting in a break down of the rigid sugar-drug matrix into fine small particles. It is believed that the milling breaks down potential drug agglomerates, which are fixated across the breaking surfaces of the rigid sugar-drug matrix, an event which can further increase the solubility of the drug in the final blend. It is further believed that higher milling intensity would increase the efficiency of the milling and, consequently, would also increase the dissolution and absorption.

The desired dissolution rate is not always defined as "as fast as possible." In certain circumstances, it is desired to control the rate at which the drug having low aqueous solubility, e.g., fenofibrate, is released by adjusting the parameters of the process of manufacture as above, or adjusting the content of the at least one at least partially amorphous sugar.

### Example 1

### (CONTROL FOR COMPARISON)

### Formulation 1 (P00266)

A fenofibrate tablet was made by conventional wet granulation based on the four steps described below. The ingredients in Table 1 were wet granulated and then compressed into tablets weighing 1368 mg. The dissolution profile of Formulation 1 in 1000 mL of 0.5% aqueous SLS solution, paddle (Apparatus II) at 50 rpm, was determined and compared with commercial versions of fenofibrate tablets ("old version" Tricor 160 mg and "newer version" Tricor 145 mg with supposedly increased dissolution and bioavailability).

**Table 1**

| (Formulation 1; about 56.5% sugar) | | |
|---|---|---|
| Ingredient | Weight (mg/tablet) | Approx.Weight Percent |
| **Part I** | | |
| Fenofibrate | 145 | 10.6 |
| Sodium Lauryl Sulfate (SLS) | 50 | 3.7 |
| Polyvinylpyrrolidone (PVP K-30) | 100 | 7.3 |
| Sucrose (SUGAT - commercially available in food industry) | 644 | 47.1 |
| Glucose monohydrate | 128.8 | 9.4 |

| **Part II** | | |
|---|---|---|
| Pregelatinized Starch (Starch 1500) | 217 | 15.9 |
| Croscarmellose Sodium (Ac-di-sol™) | 50 | 3.7 |
| Colloidal Si O₂(Aerosil™ 200) | 13 | 1 |

| **Part III** | | |
|---|---|---|
| Magnesium stearate | 20 | 1.5 |

1. The Part I ingredients were thoroughly blended.
2. The blend of Part I was granulated by adding water (approx. 0.5 cc per unit dose), granules were dried at about 65°C and milled with a small laboratory scale mill (IKA® Werke GmbH & Co.) fitted with a 0.5mm aperture screen.
3. The Part II ingredients were then blended with the granules of step 2 for about 2 minutes.
4. The Part III ingredient was then blended with the blend of step 3 for about 2 minutes. The final blend was compressed into capsule shaped tablets of 10mm/21mm dimensions.

### Examples 2-7

Examples 2-7 display the effects of manipulating the parameters discussed above including, a high weight ratio of the at least one at least partially amorphous sugar to drug; a high weight % of the "drug-sugar matrix" in the final formulation; low or high mixing temperature and short or long mixing time.

A fenofibrate tablet weighing 1368 mg was made from the ingredients listed in Table 2 by the process of the invention. Formulations 2-7 are examples of using a relatively high weight ratio (for the specified dose of 145 mg) of the at least one at least partially amorphous sugar to drug, e.g., about 6:1 1 to about 4:1; relatively high weight % of the "sugar-drug matrix" in the final formulation, e.g., about 65 to about 95 weight percent; low or high mixing temperatures; and short or long mixing time of the heated mass.

The dissolution profiles of Formulations 4 and 7 (before and after tabletting) in 1000 mL of 0.5% aqueous SLS solution, paddle (Apparatus II) at 50 rpm, were determined and compared with the dissolution profiles of commercial versions of fenofibrate tablets ("old version" Tricor 160 mg and "newer version" Tricor 145 mg) (for results see dissolution rate study below).

If the dissolution profiles of Formulations 1-3, 5 and 6 are determined, a trend of improved dissolution rate with increases in mixing time and/or mixing temperature should be observed.

**Table 2**

| Formulations 2-7 | | |
|---|---|---|
| Ratio of the at least one at least partially amorphous sugar to drug = 5.3:1 Weight % of the "sugar-drug matrix" in the final formulation = 78% | | |
| Ingredient | Weight (mg/tablet) | Approx. Weight Percent |
| **Part I** | | |
| Sucrose (SUGAT- commercially available in food industry) | 644 | 47.1 |
| Glucose | 128.8 | 9.4 |

| **Part II** | | |
|---|---|---|
| Fenofibrate | 145 | 10.6 |
| Sodium Lauryl Sulfate (SLS) | 50 | 3.7 |
| Polyvinylpyrrolidone (PVP K-30) | 100 | 7.3 |

| **Part III** | | |
|---|---|---|
| Pregelatinized Starch (Starch 1500) | 217 | 15.9 |
| Croscarmellose Sodium (Ac-di-sol™) | 50 | 3.7 |
| Colloidal Si O₂ (Aerosil^{™} 200) | 13 | 1 |

| **Part IV** | | |
|---|---|---|
| Magnesium stearate | 20 | 1.5 |

**Table 3**

| Parameter Table | | | |
|---|---|---|---|
| Formulation | X1 (vessel temp.) | X2 (blend temp.) | X3 (extra time) |
| Formulation 2 P00255A1 | 154° | 60-80° | 0 min |
| Formulation 3 P00255A2 | 154° | 60-80° | 1.5 min |
| Formulation 4 P00255A3 | 154° | 60-80° | 3 min |
| Formulation 5 P00255B1 | 170° | 90° | 0 min |
| Formulation 6 P00255B2 | 170° | 90° | 1.5 min |
| Formulation 7 P00255B3 | 170° | 90° | 3 min |

1. To the sucrose of Part 1, 322 µL of water were added. The mixture was heated until the temperature reached 125°C. At this stage the glucose of Part 1 was added to the mixture and the heating was continued until temperature reached 156°C. At this stage the solution was allowed to cool to room temperature. The amorphous sugar obtained was milled by using a Fitzmill^{™} Communitor (1000 rpm, knives forward, 0.5 mm aperture screen).
2. Powder of step 1 and ingredients of Part II were thoroughly blended. The blend was transferred into a pot heated externally to a vessel wall temperature of X1, and the blend was mixed while heated ( the blend temperature, about X2, was measured by sticking a thermometer into the blended mass). The mixing of the blend proceeded until the powder collapsed into a sticky mass in approximately 3 min, and then the sticky mass was mixed for an extra period of X3 minutes. After mixing, the mass was allowed to cool to room temperature (at approximately 20°C), and stored under dry conditions (<30% Relative Humidity) for about 1 hour. See the values of X1, X2 and X3 in the Parameter Table.
3 The rigid mass of step 2 was milled using a Fitzmill^{™} Communitor (2000 rpm, knives forward, 0.5 mm aperture screen).
4. The Part III ingredients were then mixed and then blended with the powder of step 3 for about 15 minutes.
5. The Part IV ingredient was then blended with the powder of step 4 for about 5 minutes.

The final blend was compressed into capsule shaped tablets having dimensions of 10 mm/21 mm. Tablet hardness was found to be approximately 12-20 Strong-Cobb units (SCU).

### Examples 8, 9 and 10

Examples 8-10 exemplify a low weight ratio of the at least one at least partially amorphous sugar to total drug content in the finished dosage form - high temperature; short or long mixing time.

Fenofibrate tablets comprising a relatively low weight ratio of the at least one at least partially amorphous sugar to drug in the sugar-drug matrix (for a fenofibrate dose of 145 mg), i.e., from about 0.5:1 1 to about 1.5:1, can be prepared as per the process of the invention as described in Examples 2 through 7 using less sugar and thus the ratio of sugar to fenofibrate is decreased. It is believed, however, that if a low ratio of the at least one at least partially amorphous sugar to drug is desired whilst wishing to maintain, at least partially the advantages of the invention, the preferred method would be to produce the Milled Rigid Mass, i.e. the milled sugar-drug matrix, at the same or similar ratio of fenofibrate to the at least one at least partially amorphous sugar as produced by step 3 of the above process and then to add additional fenofibrate in the blending of step 4 to the step 3 materials, and continuing the process so as to achieve a total composition with ratio of the at least one at least partially amorphous sugar to total drug between about 0.5:1 to about 3:1 amorphous sugar in the finished dosage form.

These formulations will similarly be influenced by the process conditions of temperature and time as described above when producing the hot blended mass. But overall it will be expected that those formulations comprising less amorphous sugar will have lower dissolution rates as compared to the formulations where all the fenofibrate is fixed within the sugar-drug matrix, i.e., all the fenofibrate is homogeneously dispersed in the sugar-drug matrix.

Formulations 8-10 are examples of employing low total contents of amorphous sugar. The dissolution profiles of Formulation 8-10 can be determined in 1000 mL of 0.5% aqueous SLS solution, paddle (Apparatus II) at 50 rpm,

**Table 4**

| Formulations 8, 9 and 10 | | | |
|---|---|---|---|
| Ratio of amorphous sugar to total drug = 0.66:1 (Formulation 8); 1.5:1 (Formulation 9); 2.6:1 (formulation 10), and weight % of the "sugar-drug-matrix" in the final formulation = 23.4% (Formulation 8); 42.5% (Formulation 9); and 58% (formulation 10) | | | |
| | Approx. Weight (Weight %) | | |
| Ingredient | Formulation 8 | Formulation 9 | Formulation 10 |
| **Part I** | | | |
| Sucrose | 80 (~14%) | 180 (~25.6%) | 310 (~35%) |
| Glucose | 16 (2.9%) | 36 (5.1%) | 62 (7.0%) |

| **Part II** | | | |
|---|---|---|---|
| Fenofibrate | 18 (3.2%) | 40.5 (5.8%) | 70 (7.9%) |
| Sodium Lauryl Sulfate (SLS) | 6.2 (1.1%) | 14 (2.0%) | 24 (2.7%) |
| Polyvinylpyrrolidone (PVP K-30) | 12.4 (2.2%) | 28 (4.0%) | 48 (5.4%) |

| **Part III** | | | |
|---|---|---|---|
| Fenofibrate | 127 (22.6%) | 104.5 (14.9%) | 75 (8.4%) |
| Pregelatinized Starch (Starch 1500) | 217 (38.8%) | 217 (30.9%) | 217 (24%) |
| Croscarmellose Sodium (Ac-di-sol™) | 50 (8.9%) | 50 (7.1%) | 50 (5.6%) |
| Colloidal Si O₂ (Aerosil^{™} 200) | 13 (2.3%) | 13 (1.8%) | 13 (1.5%) |

| **Part IV** | | | |
|---|---|---|---|
| Magnesium stearate | 20 (3.5%) | 20 (2.8%) | 20 (2.2%) |

1. To the sucrose of part 1 water is added (40, 90 and 155 µl for formulations 8, 9 and 10, respectively). The mixture is heated until the temperature reaches 125° C. At this stage the glucose of Part 1 is added to the mixture and the heating is continued until temperature reaches 156° C. At this stage the solution is allowed to cool to room temperature. The amorphous sugar obtained is milled by Fitzmill^{™} Communitor (1000 rpm, knives forward, 0.5 mm aperture screen).
2. Powder of step 1 and ingredients of Part II are thoroughly blended. The blend is transferred into a pot heated externally to the set temperature, the blend is heated while mixing, the blend temperature is measured by sticking a thermometer into the blended mass. The mixing proceeds until the powder collapses into a sticky mass, followed by extra predefined time of mixing the mass. After mixing, the mass is allowed to cool to room temp (~20° C), and stored under dry conditions (<30% Relative Humidity) for about 1 hour.
3 The rigid mass of step 2 is milled by using a Fitzmill^{™} Communitor (2000 rpm, knives forward, 0.5 mm aperture screen).
4. The Part III ingredients, including the remaining fenofibrate are then blended with the powder of step 3 for about 15 minutes.
5. The Part IV ingredient is then blended with the powder of step 4 for about five minutes.
6. The final blend is compressed into tablets.

### Example 11

### Formulation 11 (P-00260B3; medium ratio of amorphous sugar to drug; high temperature; long mixing time).

Fenofibrate tablets of the invention can also be prepared where the ratio of the amorphous sugar to fenofibrate in the sugar-drug matrix is lower than that in Examples 2 through 7 while ALL of the fenofibrate is fixed in the sugar-drug matrix. The formulation exemplified in Example 11 is described as having a "medium ratio" (for the specified dose of 145 mg) of the amorphous sugar to drug, i.e., having a lower weight ratio of the at least one at least partially amorphous sugar to the drug than that used in formulations exemplified by Examples 2 through 7 which are examples of high weight ratio of the at least one at least partially amorphous sugar to the drug.

Fenofibrate tablets weighing 1095 mg were made from the ingredients listed in Table 5 by the process of the invention. Formulation 11 is an examples in which a medium ratio (for the specified dose of 145 mg) of amorphous sugar to drug, i.e., about 3:1 1 to about 4:1; relatively high weight % of the "drug-sugar matrix" in the final formulation, e.g., about 65 to about 95% weight percent; high mixing temperatures and high mixing time of the heated mass were used. The dissolution profile of Formulation 11 in 1000 mL of 0.5% aqueous SLS solution, paddle (USP Apparatus II) at 50 rpm, was determined and compared with commercial versions of Fenofibrate tablets ("Old version" Tricor 160 mg and "Newer version" Tricor 145 mg ) (see dissolution rate study below).

**Table 5**

| Formulation 11 | |
|---|---|
| Ratio of amorphous sugar to drug = 3.4 :1 Weight % of the "sugar-drug-matrix" in the final formulation = 72.5% | |
| | Formulation 11 |
| Ingredient | Weight in mg/tablet (Weight %) |
| **Part I** | |
| Sucrose (SUGAT- commercial for food industry) | 416.6 (~38%) |
| Glucose | 83 (7.6%) |

| **Part II** | |
|---|---|
| Fenofibrate | 145 (13.2%) |
| Sodium Lauryl Sulfate (SLS) | 50 (4.6%) |
| Polyvinylpyrrolidone (PVP K-30) | 100 (9.1 %) |

| **Part III** | |
|---|---|
| Pregelatinized Starch (Starch 1500) | 217 (~20%) |
| Croscarmellose Sodium (Ac-di-sol™)ol | 50 (4.6%) |
| Colloidal Si O₂ (Aerosil^{™} 200) | 13 (1.2%) |

| **Part III** | |
|---|---|
| Magnesium stearate | 20 (1.8%) |

Formulation 11 was prepared using the following procedure.
1. To the sucrose of part I, 208 µL (per tablet) of water were added. The mixture was heated until the temperature reached 125° C. At this stage, the glucose of Part I was added to the mixture and the heating was continued until the temperature reached 156° C. At this stage the solution was allowed to cool to room temperature. The amorphous sugar obtained was milled by using a Fitzmill^{™} Communitor (1000 rpm, knives forward, 0.5 mm aperture screen).
2. Powder of step 1 and ingredients of Part II were thoroughly blended. The blend was transferred into a pot heated externally to 170° C, and the blend was heated while mixing the blend (the temperature, which was measured by inserting the thermometer directly into the blended mass, was about 90°C). The mixing proceeded until the powder collapsed into a sticky mass (~3 minutes), followed by extra 3 minutes of mixing the mass. After mixing, the mass was allowed to cool to room temperature (about 16° C), and stored under dry conditions (<30% relative humidity for about 1 hour).
3 The rigid mass of step 2 was milled by using a Fitzmill^{™} Communitor (2000 rpm , knives forward, 0.5 mm aperture screen).
4. The Part III ingredients were then mixed and then blended with the powder of step 3 for about 15 minutes.
5. The Part IV ingredient was then blended with the powder of step 4 for about five minutes.
6. The final blend was compressed into capsule shaped tablets having dimensions of 10 mm/21 mm.

Tablet hardness was found to be ~ 12-20 Strong-Cobb units (SCU).

### Example 12 (P-00268)

### Formulations 12 - Different type of amorphous sugar, high ratio of amorphous sugar to drug; high weight % of the "drug-sugar matrix" in the final formulation

Formulations of the invention can also be made from amorphous sugar that has been prepared without the use of glucose.

Fenofibrate tablets weighing 1239 mg were made from the ingredients listed in Table 6 by the process of the invention. Formulation 12 is an example of using a relatively high ratio (for the specified dose of 145 mg) of amorphous sugar to drug, e.g., about 6:1 to about 4:1; a relatively high weight % of the "drug-sugar matrix" in the final formulation, e.g., about 65 to about 95% weight percent; and a mixing time of 2 minutes of the heated mass. A major difference between Formulation 12 and Formulations 2-7 is that Formulation 12 used only sucrose as a source for the preparation of the amorphous sugar, while Formulations 2-7 used sucrose and glucose. The dissolution profile of Formulation 12 in 1000 mL of 0.5% aqueous SLS solution, paddle (Apparatus II) at 50 rpm, was determined and compared with commercial versions of Fenofibrate tablets ("Old version" Tricor 160 mg and "Newer version" Tricor 145 mg) (see dissolution rate study below).

**Table 6**

| Formulation 12 | | |
|---|---|---|
| Ratio of amorphous sugar to drug = 4.4 : 1 Weight % of the "sugar-drug matrix" in the final formulation = 75.7% | | |
| Ingredient | Weight (mg/tablet) | Weight % |
| **Part I** | | |
| Sucrose (SUGAT - comerical for food industry) | 644 | ~52% |

| **Part II** | | |
|---|---|---|
| Fenofibrate | 145 | 11.7% |
| Sodium Lauryl Sulfate (SLS) | 50 | 4.0% |
| Polyvinylpyrrolidone (PVP K-30) | 100 | 8.0% |

| **Part III** | | |
|---|---|---|
| Pregelatinized Starch (Starch 1500) | 217 | 17.5% |
| Croscarmellose Sodium (Ac-di-sol™) | 50 | 4.0% |
| Colloidal Si O₂ (Aerosil^{™} 200) | 13 | 1.0% |

| **Part IV** | | |
|---|---|---|
| Magnesium stearate | 20 | 1.5% |

1. To the sucrose of part I, 322µL of water were added. The mixture was heated until the temperature reached 156° C. At this stage the solution was allowed to cool to room temperature. The amorphous sugar obtained was milled by using a Fitzmill^{™} Communitor (1000 rpm, knives forward, 0.5 mm aperture screen).
2. Powder of step 1 and ingredients of Part II were thoroughly blended. The blend was transferred into a pot heated externally to 170° C, and the blend was heated while mixing (the blend temperature was measured by sticking a thermometer into the blended mass and was about 90° C). The mixing proceeded until the powder collapsed into a sticky mass (~3 min), followed by extra 2 minutes of mixing the mass. After mixing the mass was allowed to cool to room temp (about 20° C), and stored under dry conditions (<30% RH for about 1 hour).
3 The rigid mass of step 2 was milled by a small laboratory scale mill (IKA® Werke GmbH & Co.) fitted with a 0.5 mm aperture screen.
4. The Part III ingredients were then mixed and then blended with the powder of step 3 for about 15 minutes.
5. The Part IV ingredient was then blended with the powder of step 4 for about five minutes.
6. The lubricated granules were compressed into capsule shaped tablets having dimensions of 10 mm/21 mm.

Tablet hardness was found to be ~ 12-20 Strong-Cobb units (SCU).

### Example 13 (P00267)Formulations 13 (comparative; using entirely crystalline sugar instead of amorphous sugar -- high temperature; long mixing time)

Whereas the formulations of the invention are manufactured using a process that involves heating fenofibrate and amorphous sugar, a comparative example was performed wherein this same process was performed where the sugar used was in crystalline form and thus the preparatory step of producing the amorphous sugar was absent. Fenofibrate tablets weighing 1368 mg were made from the ingredients listed in Table 7. Formulations 13 is an example of using a relatively high content of crystalline sugar from about 50 weight % to about 99 weight %, employing high mixing temperatures and long mixing time (of the heated mass). The dissolution profile of Formulation 13 in 1000 mL of 0.5% aqueous SLS solution, paddle (Apparatus II) at 50 rpm was tested and compared with commercial versions of Fenofibrate tablets ("Old version" Tricor 160 mg and "Newer version" Tricor 145 mg) (see dissolution rate study, below).

**Table 7**

| (Formulation 13) | | |
|---|---|---|
| Ingredient | Weight (mg/tablet) | Weight Percent |
| **Part I** | | |
| Sucrose (SUGAT - comerical for food industry) | 644 | 47.1 |
| Glucose | 128.8 | 9.4 |
| Fenofibrate | 145 | 10.6 |
| Sodium Lauryl Sulfate (SLS) | 50 | 3.7 |
| Polyvinylpyrrolidone PVP k-30 | 100 | 7.3 |

| **Part II** | | |
|---|---|---|
| Pregelatinized Starch (Starch 1500) | 217 | 15.9 |
| Croscarmellose Sodium (Ac-di-sol™) | 50 | 3.7 |
| Colloidal Si O₂ (Aerosil^{™} 200) | 13 | 1 |

| **Part III** | | |
|---|---|---|
| Magnesium stearate | 20 | 1.5 |

1. Ingredients of Part I were thoroughly blended. The blend was transferred into a pot heated externally to 170° C, and the blend was heated while mixing. The mixing was preceded for 5 minutes. After mixing the mass was allowed to cool to room temperature (about 20° C).
2 The mass of step 1 was milled by using a small laboratory scale mill (IKA® Werke GmbH & Co.), fitted with a 0.5 mm aperture screen.
3. The Part II ingredients were then blended with the powder of step 2 for about fifteen minutes.
4. The Part III ingredient was then blended with the powder of step 3 for about five minutes.
5. The lubricated blend was compressed into capsule shaped tablets of dimensions 12.0 mm x 5.5 mm.

Tablet hardness was found to be 9 Strong-Cobb units.

### Example 14 (K-35211)

### Formulation 14 - Use of formulated granulate as a drug source in the "drug-sugar matrix", relatively high ratio of amorphous sugar to drug (for the specified dose of 145 mg), high weight % of the "sugar-drug- matrix" in the final formulation.

A fenofibrate tablet weighing 1444 mg was made from the ingredients listed in Table 8 by the process of the invention. Formulation 14 is example of using a formulated granulate of fenofibrate as a drug source in the process for preparing the sugar-drug matrix. Also this example used a relatively high ratio (for the specified dose of 145 mg) of the amorphous sugar to drug, e.g., about 6:1 to about 4:1; and a relatively high weight % of the "sugar-drug-matrix" in the final formulation, e.g., about 65 to about 95 weight %.

Also this example used high mixing temperature and 1 min mixing time of the heated mass. The dissolution profiles of Formulation 14 in 1000 mL of 0.5% aqueous SLS solution, paddle (Apparatus II) at 50 rpm, were determined and compared with that of the commercially available "newer version" Tricor 145 and Teva's formulation k-29740 (bioequivalent to Tricor 160 mg ) (for results see dissolution rate study below).

**Table 8**

| Ingredient | Weight (mg/tablet) | Approx.W eight Percent |
|---|---|---|
| **Part I** | | |
| Sucrose | 453.0 | 31.4 |
| Glucose | 90.6 | 6.3 |

| **Part II** | | |
|---|---|---|
| "Granulated Fenofibrate" see below | 682 | 47.2 |

| **Part III** | | |
|---|---|---|
| Colloidal Si O₂ (Aerosil^{™} 200) | 50 | 3.5 |

| **Part IV** | | |
|---|---|---|
| Sodium Starch Glycolate (Primojel^{™}) | 42 | 2.9 |
| Croscarmellose Sodium (Ac-di-Sol™) | 42 | 2.9 |
| Croscarmellose Sodium (Ac-di-sol™) | 42 | 2.9 |

| **Part V** | | |
|---|---|---|
| Magnesium stearate NF | 42.4 | 2.9 |

1. To the Sucrose of part 1, 227 µL of water were added. The mixture was heated until the temperature reached 125° C. At this stage the glucose of part 1 was added to the mixture and the heating was continued until temperature reached 156° C. At this stage the solution was allowed to cool to room temperature. The amorphous sugar obtained was milled by Fitzmill^{™} Communitor (medium speed, knives forward, 1.65 mm aperture screen).
2. Powder of stage 1 and ingredients of Part II were thoroughly blended. The blend was transferred into a pot heated externally to 160° C vessel wall temperature, and the blend was mixed while heated (the blend temperature was measured by sticking thermometer into the blended mass and was about 90° C. The mixing proceeded until the powder collapsed into a sticky mass (approx. 1min), followed by an extra 2-3minute of mixing the mass. After mixing, the mass was allowed to cool to room temp (approximately 20°C), and stored under dry conditions (<30% Relative Humidity) for about 1 hour.
3. The Part III ingredient was added to the rigid mass of stage 2 and together were milled with a Fitzmill^{™} Communitor ( medium speed, knives forward, 2 to 0.5 mm aperture screen ).
4. The Part IV ingredients were then mixed and then blended with the powder of stage 3 for about 15 minutes.
5. The Part V ingredient was then blended with the powder of stage 4 for about 15 minutes.
6. The lubricated final blend was compressed into capsule shaped tablets.

### Preparation of the "granulated fenofibrate" used in Part II of Table 8

The "granulated fenofibrate" was prepared according to the following procedures using the ingredients of Table 9.

**Table 9**

| Ingredient | Weight (mg/tablet) | Approx.Weight Percent |
|---|---|---|
| **Part I** | | |
| Fenofibrate | 145 | 21 |
| Polyvinylpyrrolidone (PVP K-30) | 16 | 2.3 |
| Polyvinylpyrrolidone (PVP K-25) | 38 | 5.6 |
| Sodium Starch Glycolate (Primojel) | 44 | 6.5 |
| Croscarmellose Sodium (Ac-di-sol™) | 44 | 6.5 |
| Crospovidone NF | 44 | 6.5 |
| Microcrystaline Cellulose (Avicel) | 127 | 18.6 |

| **Part II** | | |
|---|---|---|
| Sodium Lauryl Sulfate (SLS) | 14 | 2.1 |
| Lactose | 192 | 28.2 |

| **Part III** | | |
|---|---|---|
| Colloidal SiO₂ Aerosil 200 | 7 | 1.0 |

| **Part IV** | | |
|---|---|---|
| Pruv (Sodium Stearyl Fumarate) | 11 | 1.6 |

1. Part I ingredients were thoroughly blended.
2. Lactose of Part II was dissolved in 192 mg of water heated to about 70°C.
3. SLS of Part II was dissolved in about 10 mg of water.
4. The blend of stage 1 was granulated by adding the lactose and SLS solutions of stage 2 and 3.
5. The granules were dried in a fluidized bed dryer (inlet air 55° C, outlet air not more than 40° C).
6. Aerosil of Part III was blended with the granules of stage 5 and then milled by Fitzmill^{™} ( fitted with a 0.5 mm aperture screen).
7. The Part IV ingredients were then blended with the granules of stage 6 for about 2 minutes.
8. The final blend was compressed into tablets.
9. The tablets were milled with a Fitzmill^{™} Communitor ( knives forward, 0.5 mm aperture screen).

### Dissolulation Rate Study

The dissolution properties of the blends or tablets prepared according to Formulation 1 (control), Formulations 4, 7, 11 and 12 (invention), Formulation 13 (comparative; crystalline sugar), Formulation 14 (invention), K-29740 (control - used as an alternative to Tricor 160 mg), Tricor 14.5 mg and Tricor 160 mg were determined using 1000 cc of 0.5% aqueous SLS solution, 50 rpm paddle, 37° C for up to 60 minutes. The results are presented in Tables 10-13 and illustrated in Fig. 1-3.

**Table 10**

| Time (min) | Tricor 145mg | Tricor 160mg | Formulation 1 tablets. (P00266) | Formulation 13 tablets. (P00267) | Formulation 12 tablets. (P00268) | Formulation 11 tablets. (P00260B3) |
|---|---|---|---|---|---|---|
| 0 | 0.0 | 0.0 | 0.0 | 0.0 | 0.0 | 0.0 |
| 10 | 24.3 | 22.5 | 14.1 | 33.2 | 32.5 | 33.4 |
| 20 | 56.8 | 31.0 | 40.5 | 62.6 | 65.6 | 52.7 |
| 30 | 61.5 | 43.6 | 41.6 | 70.6 | 81.3 | 85.0 |
| 40 | 86.4 | 60.2 | 60.8 | 87.6 | 91.5 | 71.3 |
| 60 | 67.0 | 53.7 | 67.6 | 98.9 | 83.9 | 68.6 |

**Table 11**

| Time (min) | Tricor 145mg | Tricor 160mg | Formulation 4 final Blend (P00255A3) | Formulation 7 final Blend (P00255B3) |
|---|---|---|---|---|
| 0 | 0.0 | 0.0 | 0.0 | 0.0 |
| 10 | 32.3 | 30.9 | 47.5 | 55.7 |
| 20 | 70.3 | 40.9 | 62.2 | 68.5 |
| 30 | 67.8 | 48.2 | 68.4 | 68.0 |
| 40 | 87.7 | 61.1 | 80.6 | 81.6 |
| 60 | 94.1 | 73.3 | 81.6 | 75.4 |

**Table 12**

| Time (min) | Tricor 145mg | Tricor 160mg | Formulation 4 tablets. (P00255A3) | Formulation 7 tablets. (P00255B3) |
|---|---|---|---|---|
| 0 | 0.0 | 0.0 | 0.0 | 0.0 |
| 10 | 52.4 | 30.4 | 39.8 | 42.9 |
| 20 | 88.8 | 58.9 | 67.0 | 73.8 |
| 30 | 92.7 | 68.8 | 77.9 | 87.8 |
| 60 | 83.3 | 72.1 | 81.3 | 88.3 |

**Table 13**

| Time (min) | Tricor 145mg | K-29740 | Formulation 14 tablets. (K-35211) |
|---|---|---|---|
| 0 | 0.0 | 0.0 | 0.0 |
| 10 | 48.5 | 37.2 | 39.6 |
| 20 | 64.0 | 47.7 | 55.2 |
| 30 | 66.6 | 55.5 | 58.4 |
| 40 | 67.8 | 51.6 | 52.9 |
| 60 | 66.1 | 56.9 | 52.4 |

## Claims

1. A process for making a pharmaceutical composition of a drug having low aqueous solubility, the process comprising
(A) fixing the drug in a strong matrix comprising at least one at least partially amorphous sugar to obtain a sugar-drug matrix; and
(B) milling the sugar-drug matrix to obtain a milled sugar-drug matrix as the pharmaceutical composition, the composition being optionally further processed into a pharmaceutical formulation

2. The process of claim 1, wherein the at least one sugar is substantially amorphous.

3. The process of claim 1 or claim 2, wherein step (A) is performed by heating a mixture of the drug and the at least one at least partially amorphous sugar to obtain the sugar-drug matrix.

4. The process of any preceding claim, wherein step (A) is performed by heating a mixture of the drug and at least one sugar with cooling to obtain the sugar-drug matrix.

5. The process of claim 3 or 4, wherein the heating is followed by cooling to obtain the sugar-drug matrix.

6. The process of any of claims 3 to 5, wherein the heating is accompanied with mixing of the drug and the at least one at least partially amorphous sugar or the at least one sugar.

7. The process of any preceding claim, wherein the sugar-drug matrix obtained in step (A) is a substantially homogeneous dispersion of the drug in the strong matrix.

8. The process of any preceding claim, wherein the duration and conditions of said milling in step (B) are such that the drug in said milled matrix has a desired dissolution rate.

9. The process of any preceding claim, wherein step (A) is performed as step (a) below and step (B) is performed as step (b) below, such that the process comprises
(a) mixing and heating the drug with the following excipient(s) to form a sugar-drug matrix, or mixing the drug with the following excipient(s) followed by heating to form a sugar-drug matrix comprising at least the drug and at least one at least partially amorphous sugar:(i) at least one sugar or at least one at least partially amorphous sugar in a weight ratio of from about 1:10 to about 1000:1 as compared to the drug,
(ii) optionally a surface active agent in an amount of from about 1 to about 5 weight %, and
(iii) optionally a dispersing agent in an amount of from about 1 to about 10 weight %; and
(b) milling the sugar-drug matrix to obtain a milled sugar-drug matrix as the pharmaceutical formulation,
wherein the weight % is based on the total weight of the pharmaceutical formulation.

10. The process of claim 9, wherein the weight ratio of the at least one at least partially amorphous sugar to the drug in the sugar-drug matrix in step (a) is from about 1.5:1 to about 10:1, preferably from about 2:1 to about 8:1, more preferably from about 3:1 to about 6:1.

11. The process of claims 9 or 10, wherein the surface active agent is used in step (a) and is sodium lauryl sulfate.

12. The process of any of claims 9 to 11, wherein the dispersing agent is used in step (a) and is polyvinyl pyrrolidone.

13. The process of any of claims 9 to 12, wherein the duration and conditions of said milling in step (b) are such that the drug in said milled matrix has a desired dissolution rate

14. The process of any of claims 1 to 8, wherein step (A) is performed by conducting steps (a)-(c) below and step (B) is performed by conducting step (d) below, such that the process comprises
(a) blending the drug, the at least one at least partially amorphous sugar in powder form, the optional surface active agent and the optional dispersing agent,
(b) mixing the blend, while at elevated temperature until a "smooth" mixture is obtained by controlling the time and intensity of mixing;
(c) cooling the "smooth" mixture to ambient temperature or below room temperature to obtain a sugar-drug matrix; and
(d) milling the sugar-drug matrix obtained in step (c) optionally with at least one glident to obtain a milled sugar-drug matrix as the pharmaceutical formulation.

15. The process of claim 14, wherein the drug used in step (a) is a drug in a formulated granulate.

16. The process of claim 15, wherein the formulated granulate containing the drug is obtained by conventional wet granulation or conventional dry granulation process.

17. The process of claim 15, wherein the formulated granulate containing the drug is obtained by wet granulation using a solution of lactose as a binder solution.

18. A process for making the formulated granulate of claim 15 for pharmaceutical compositions, comprising
(a) combining an active pharmaceutical ingredient having poor water solubility, a solution of at least one pharmaceutically acceptable sugar and, optionally, at least one pharmaceutically acceptable excipient other than the at least one pharmaceutically acceptable sugar to form a mixture, wherein the active pharmaceutical ingredient has a water solubility of less than about 20 mg per ml of water, and wherein the solution comprises the at least one pharmaceutically acceptable sugar and at least one solvent;
(b) removing the at least one solvent from the mixture; and
(c) comminuting the product of step (b) to obtain the formulated granulate.

19. The process of any of claims 14 to 17, wherein the drug and the at least one at least partially amorphous sugar are blended with a surface active agent and dispersing agent in step (a).

20. The process of claim 19, wherein the surface active agent is sodium lauryl sulfate and the dispersing agent is polyvinyl pyrrolidone.

21. The process of any of claims 14 to 17 or claim 19, wherein the blend in step (b) is heated to a temperature ranging from about 50° C to about 200° C.

22. The process of claim 21, wherein the blend in step (b) is heated to a temperature ranging from about 70° C to about 120° C.

23. The process of any of claims 14 to 17 and claims 19 to 22, further comprising, after step (d),
(e) making a blend with a method comprising the following steps:
(e1) heating the milled sugar-drug matrix with mixing until a "smooth" mixture is obtained;
(e2) cooling the "smooth" mixture to room temperature or below room temperature to obtain a cooled matrix;
(e3) milling the cooled matrix obtained in step (e2) to obtain a milled matrix as a blend containing a powder; and
(e4) optionally repeating steps (e1) to (e3) until the powder in the milled matrix reaches a desired rate of dissolution;
(f) optionally adding at least one pharmaceutically acceptable excipient other than the at least one pharmaceutically acceptable sugar, surface active agent and dispersing agent to the blend obtained from step (e) in order to improve the handling and/or compression of the blend; and optionally
(g) tabletting the blend or filling the blend into capsules or sachets.

24. The process of claim 23, wherein the milled sugar-drug matrix in step (e1) is heated to a temperature ranging from about 50° C to about 200° C.

25. The process of claim 24, wherein the milled sugar-drug matrix in step (e1) is heated to a temperature ranging from about 70° C to about 120° C.

26. The process of any of claims 23 to 25, wherein the at least one pharmaceutically acceptable excipient in step (f) is selected from diluents, disintegrants, lubricants and glidants.

27. The process of any of claims 1 to 8, wherein the weight ratio of the at least one at least partially amorphous sugar and the active drug in the sugar-drug matrix is between about 1:10 to about 1000:1, preferably about 1.5:1 to about 10:1, more preferably about 2:1 to about 8:1, most preferably about 3:1 to about 6:1.

28. The process of any preceding claim, wherein the amount of the sugar-drug matrix in the final formulation is from about 10 weight% to about 99 weight %, preferably from about 30 weight% to about 95 weight%, most preferably from about 60 weight% to about 90 weight%.

29. The process of any preceding claim, wherein the amount of the drug in the pharmaceutical formulation is about 1 weight% to about 95 weight% based on the total weight of the pharmaceutical formulation.

30. The process of claim 29 where the dose of the active drug is about 145mg.

31. The process of any preceding claim, wherein the drug is selected from fenofibrate, bicalutamide, atorvastatin, fluvastatin, simvastatin, paclitaxel, aripiprazol, glyburide, ezetimibe, oxcarbazepine, meloxicam, celecoxib, rofecoxib, valdecoxib and raloxifene.

32. The process of claim 32, wherein the drug is fenofibrate.

33. The process of any preceding claim, wherein the at least one at least partially amorphous sugar is made from sucrose and glucose.

34. The process of any of claims 1 to 32, wherein the at least one at least partially amorphous sugar is made from sucrose.

35. The process of any of claims 1 to 32, wherein said sugar-drug matrix comprises sucrose and glucose.

36. The process of claim 35, wherein the process for preparation of at least one at least partially amorphous sugar comprises:
a) heating a mixture of sucrose and water up to about 125°C to form a hot mixture;
b) adding glucose to the hot mixture;
c) heating the mixture obtained from step b) up to about 156°C; and
d) cooling the mixture obtained from step c) to about room temperature and optionally storing under dry conditions to obtain the at least one at least partially amorphous sugar.

37. The process of claim 36, wherein the weight ratio of sucrose to water is about 1:0.5.

38. The process of claim 36 or 37, wherein the glucose comprises about 20% of the weight of sucrose.

39. A pharmaceutical formulation prepared by the process of any preceding claim.

40. A pharmaceutical formulation comprising particles of a strong sugar-drug matrix, wherein the matrix comprises a drug of low aqueous solubility dispersed in at least one at least partially amorphous sugar.

41. The pharmaceutical formulation of claim 40, wherein the sugar-drug matrix is a homogeneous dispersion of the drug in particulate form in the strong matrix.

42. The pharmaceutical formulation of claims 40 or 41, wherein the drug is selected from fenofibrate, bicalutamide, atorvastatin, fluvastatin, simvastatin, paclitaxel, aripiprazol, glyburide, ezetimibe, oxcarbazepine, meloxicam, celecoxib, rofecoxib and valdecoxib,

43. The pharmaceutical formulation of any of claims 40 to 42, wherein the at least one at least partially amorphous sugar is made from sucrose and glucose.

44. The pharmaceutical formulation of any of claims 40 to 42, wherein the at least one at least partially amorphous sugar is made from sucrose.

45. The pharmaceutical formulation of any of claims 40 to 44, wherein the weight ratio of the at least one at least partially amorphous sugar and the active drug in the sugar drug matrix is about 3:1 to about 6:1.

46. The pharmaceutical formulation of any of claims 40 to 45, wherein the amount of the sugar-drug matrix in the final formulation is from about 10 weight% to about 99 weight %, preferably from about 30 weight% to about 95 weight%, most preferably from about 60 weight% to about 90 weight%.

47. The pharmaceutical formulation of any of claims 40 to 46, wherein the active drug is fenofibrate and the dose is about 145 mg.
